# FASCICULE DE BREVET EUROPEEN

(11) **EP 2 448 594 B1**
(45) Date de publication et mention de la délivrance du brevet: **28.02.2018**
(21) Numéro de dépôt: 10745672.5
(22) Date de dépôt: 29.06.2010
(51) Int. Cl.: A61K 38/17, A61P 35/00, A61P 37/00, A61K 45/06, A61K 38/48

(54) **LA DERMASEPTINE B2 COMME INHIBITEUR DE LA CROISSANCE TUMORALE**
DERMASEPTIN B2 ALS TUMORWACHSTUMSHEMMER
DERMASEPTIN B2 USED AS AN INHIBITOR OF THE GROWTH OF A TUMOR

(30) Priorité: 01.07.2009 FR 0954505
(43) Date de publication de la demande: 09.05.2012
(73) Titulaire: Centre National de la Recherche Scientifique (C.N.R.S.), 75016 Paris (FR); Université Pierre et Marie Curie (Paris 6), 75005 Paris (FR); Université Paris-Est Créteil Val de Marne, 94010 Creteil Cedex (FR)
(72) Inventeur: DELBE, Jean, F-92410 Ville D'avray (FR); AMICHE, Mohamed, F-77186 Noisiel (FR); LADRAM, Ali, F-95120 Ermont (FR); GALANTH, Cécile, F-75020 Paris (FR); NICOLAS, Pierre, F-27950 Villez-sous-Bailleul (FR); HAMMA, Yamina, F-94270 Le Kremlin Bicetre (FR); VAN ZOGGEL, Johanna Allegonda Anna, F-94400 Vitry Sur Seine (FR); COURTY, José, F-94440 Villecresnes (FR)
(74) Mandataire: Lavoix
(86) Numéro de dépôt international: PCT/FR2010/051347
(87) Numéro de publication internationale: WO 2011/001097

(56) Documents cités:
- FR-A1- 2 735 983
- US-A1- 2007 207 209
- US-B1- 6 440 690
- AMICHE M ET AL: "Molecular Cloning of a cDNA Encoding the Precursor of Adenoregulin from Frog Skin: Relationships with the Vertebrate Defensive Peptides, Dermaseptins" BIOCHEMICAL AND BIOPHYSICAL RESEARCH COMMUNICATIONS, ACADEMIC PRESS INC. ORLANDO, FL, US, vol. 191, no. 3, 31 mars 1993 (1993-03-31) , pages 983-990, XP024767733 ISSN: 0006-291X [extrait le 1993-03-31]

### SEQUENCE LISTING

<110> CNRS
<120> LA DERMASEPTINE B2 COMME INHIBITEUR DE LA CROISSANCE TUMORALE
<130> BET 10P0997
<150> FR0954505
   <151> 2009-07-01
<160> 3
<170> PatentIn version 3.5
<210> 1
   <211> 33
   <212> PRT
   <213> Phyllomedusa bicolor
<400> 1
   Gly Leu Trp Ser Lys Ile Lys Glu Val Gly Lys Glu Ala Ala Lys Ala
   1               5                   10                  15
   Ala Ala Lys Ala Ala Gly Lys Ala Ala Leu Gly Ala Val Ser Glu Ala
               20                  25                  30
   Val
<210> 2
   <211> 81
   <212> PRT
   <213> Phyllomedusa bicolor
<400> 2
   Met Ala Phe Leu Lys Lys Ser Leu Phe Leu Val Leu Phe Leu Gly Leu
   1               5                   10                  15
   Val Ser Leu Ser Ile Cys Glu Glu Glu Lys Arg Glu Asn Glu Asp Glu
               20                  25                  30
   Glu Glu Gln Glu Asp Asp Glu Gln Ser Glu Met Lys Arg Gly Leu Trp
           35                  40                  45
   Ser Lys Ile Lys Glu Val Gly Lys Glu Ala Ala Lys Ala Ala Ala Lys
       50                  55                  60
   Ala Ala Gly Lys Ala Ala Leu Gly Ala Val Ser Glu Ala Val Gly Glu
   65                  70                  75                  80
   Gln
<210> 3
   <211> 380
   <212> DNA
   <213> Phyllomedusa bicolor
<400> 3
   tccggcactt tctgaattac aagaccagac atggctttcc ttaagaaatc tcttttcctt      60
   gtactattcc ttggattggt ctctctttct atctgcgaag aagagaaaag agaaaatgaa     120
   gatgaggagg aacaagaaga tgacgagcaa agtgaaatga agagagggct ttggagtaaa     180
   ataaaagaag taggaaaaga agcagcaaaa gctgcagcaa aagctgcagg aaaagcggct     240
   ttaggtgcag tttctgaggc ggtgggagag caataaatat aagaaaatgt aaaatcaaat     300
   tgctcttaac agagcaatta tcaatatttg tgccaaacct ttattaaagc aaattgaact     360
   gaaaaaaaaa aaaaaaaaaa                                                 380

## Description

La présente invention concerne l'utilisation de la dermaseptine B2 pour inhiber la prolifération et/ou la croissance cellulaire dans le traitement ou la prévention d'une tumeur. La thérapie actuelle du cancer repose sur la radiothérapie, la chirurgie, parfois très invalidante et/ou l'utilisation de drogues anti-cancéreuses bloquant les mitoses. Ces thérapies sont souvent très agressives ce qui peut limiter leurs utilisations. Une autre voie de traitement envisagée est l'immunothérapie qui consiste à administrer des substances qui vont stimuler les défenses immunitaires de l'organisme. Néanmoins, les patients souffrant de cancers, et en particulier des cancers métastatiques, ne répondent pas toujours ou répondent mal à l'immunothérapie. Malgré d'importantes recherches dans le monde, il n'existe actuellement pas de thérapie universelle contre cette pathologie.

En plus d'une prolifération incontrôlée des cellules tumorales, le développement durable d'une tumeur est toujours associé à une angiogenèse. Par conséquent, la suppression ou l'inhibition des facteurs induisant l'angiogenèse doit conduire à une régression de la croissance tumorale et ceci quel que soit le type de tumeur.

Aujourd'hui, plusieurs sociétés développent dans le traitement du cancer ou des maladies prolifératives, une stratégie anti-angiogenèse basée par exemple soit sur des inhibiteurs de facteurs angiogènes (ou de leur récepteurs quand ceux-ci sont identifiés), soit en induisant des micro thromboses vasculaires en utilisant la cellule endothéliale comme ancrage des catalyseurs de la thrombose, soit encore en utilisant des agents peptidiques qui inhibent l'angiogenèse par des mécanismes pas toujours identifiés. Ces approches n'ont pas encore débouché sur des résultats clairs et il apparaît que les inhibiteurs basés sur le blocage d'une seule voie de l'angiogenèse induisent des effets rebonds aggravants. Ces résultats conduisent actuellement ces sociétés à proposer des cocktails d'inhibiteurs permettant d'espérer une destruction radicale et simultanée des vaisseaux et de cellules tumorales.

Il y a donc actuellement un besoin de nouveaux inhibiteurs de l'angiogenèse, qui de ce fait inhibent la croissance et/ou la prolifération des cellules.

Les inventeurs ont maintenant démontré que, de manière surprenante, la dermaseptine B2, extraite des sécrétions de peau de la grenouille sud américaine du genre *Phyllomedusa bicolor* et connue comme agent anti-microbien, était capable d'inhiber d'une part la prolifération des cellules tumorales et d'autre part l'angiogenèse.

Le brevet américain US 6,440,690 décrit l'utilisation de peptides, notamment de la dermaseptine, pour le traitement de maladies infectieuses ou du cancer, en stimulant le système immunitaire de l'hôte. Plus précisément, ces peptides stimulent le système immunitaire en activant les cellules des lignées de monocytes/macrophages et/ou d'autres cellules lymphoïdes. En revanche, il n'est pas suggéré que la dermaseptine B2 pourrait inhiber la croissance et/ou la prolifération cellulaire.

### Description de l'invention

La présente invention concerne ainsi un peptide isolé comprenant, ou consistant en, une séquence d'acides aminés choisie dans le groupe constitué de
- la séquence de la dermaseptine B2, la séquence du précurseur de la dermaseptine B2;
- une séquence d'acides aminés ayant au moins 80% d'identité avec les séquences de la dermaseptine B2 ou du précurseur de la dermaseptine B2; et
- un fragment de ces séquences;
sous réserve que le peptide isolé inhibe la croissance et/ou la prolifération cellulaire,
pour son utilisation pour inhiber la croissance et/ou la prolifération cellulaire dans le traitement ou la prévention d'un désordre prolifératif qui est une tumeur.

La présente description décrit un peptide isolé comprenant, ou consistant en, une séquence d'acides aminés choisie dans le groupe constitué de
- la séquence de la dermaseptine B2, la séquence du précurseur de la dermaseptine B2;
- une séquence d'acides aminés ayant au moins 80% d'identité avec les séquences de la dermaseptine B2 ou du précurseur de la dermaseptine B2; et
- un fragment de ces séquences,
sous réserve que le peptide isolé inhibe la croissance et/ou la prolifération cellulaire, pour son utilisation, de préférence pour inhiber la croissance et/ou la prolifération cellulaire, dans le traitement ou la prévention d'un désordre prolifératif, d'une lésion oculaire ou d'une maladie auto-immune.

La présente invention concerne également une molécule chimère comprenant au moins un peptide tel que défini ci-dessus, dans laquelle ledit peptide est lié à :
a) un composé thérapeutique utile pour le traitement de désordres prolifératifs;
b) une enzyme capable de convertir une molécule en un composé thérapeutique utile pour le traitement de désordres prolifératifs, l'enzyme étant sélectionnée parmi les membres de la famille des métalloprotéinases matricielles, l'urokinase ou la plasmine; ou
c) une molécule porteuse,
pour son utilisation pour inhiber la croissance et/ou la prolifération cellulaire dans le traitement ou la prévention d'un désordre prolifératif qui est une tumeur.

La présente description décrit également une molécule chimère comprenant au moins un peptide tel que défini ci-dessus, dans laquelle ledit peptide est lié à :
a) un composé thérapeutique utile pour le traitement de désordres prolifératifs;
b) une enzyme capable de convertir une molécule en un composé thérapeutique utile pour le traitement de désordres prolifératifs; ou
c) une molécule porteuse.

La présente description décrit d'autre part un procédé de production d'une molécule chimère telle que définie ci-dessus, comprenant :
a) la synthèse d'un peptide tel que défini ci-dessus par voie chimique; et
b) la conjugaison dudit peptide avec un composé choisi parmi :
   i) un composé thérapeutique utile pour le traitement de désordres prolifératifs;
   ii) une enzyme capable de convertir une molécule en un composé thérapeutique utile pour le traitement de désordres prolifératifs; et
   iii) une protéine porteuse.

Un autre objet de la présente invention concerne un acide nucléique comprenant, ou consistant en, une séquence codant pour un peptide tel que défini ci-dessus, ou un vecteur comprenant cet acide nucléique, dans lequel ledit acide nucléique est lié de manière fonctionnelle à un ou des élément(s) permettant l'expression dudit peptide, pour leur utilisation pour inhiber la croissance et/ou la prolifération cellulaire dans le traitement ou la prévention d'un désordre prolifératif qui est une tumeur.

La description décrit également un acide nucléique comprenant, ou consistant en, une séquence codant pour un peptide tel que défini ci-dessus, et un vecteur comprenant cet acide nucléique dans lequel l'acide nucléique est lié de manière fonctionnelle à un ou des élément(s) permettant l'expression dudit peptide, pour leur utilisation, de préférence pour inhiber la croissance et/ou la prolifération cellulaire, dans le traitement ou la prévention d'un désordre prolifératif, d'une lésion oculaire ou d'une maladie auto-immune.

La description décrit également une composition pharmaceutique comprenant un peptide tel que défini ci-dessus ou une molécule chimère telle que définie ci-dessus, ainsi qu'une composition pharmaceutique comprenant un acide nucléique tel que défini ci-dessus ou un vecteur tel que défini ci-dessus. Ces compositions peuvent en outre comprendre un second composé thérapeutique utile pour le traitement de désordres prolifératifs, de lésions oculaires ou de maladies auto-immunes.

La présente invention concerne également un peptide tel que défini ci-dessus ou une molécule chimérique telle que définie ci-dessus, en combinaison avec un second composé thérapeutique utile pour le traitement de tumeurs, pour leur utilisation dans le traitement ou la prévention d'une tumeur.

### Dermaseptine B2

Par "dermaseptine B2" ou "adénoréguline", on entend ici un peptide de 33 acides aminés, qui a été isolée pour la première fois à partir de sécrétions de peau d'une grenouille sud-américaine du genre *Phyllomedusa bicolor* (Daly et al. (1992) Proc. Natl. Acad. Sci. USA 89:10960-10963). La séquence en acides aminés de ce peptide correspond à la séquence GLWSKIKEVGKEAAKAAAKAAGKAALGAVSEAV (SEQ ID NO: 1). Ce peptide est exprimé sous la forme d'un précurseur, la préproadénoréguline, qui est constitué de 81 acides aminés et dont la séquence est la suivante : MAFLKKSLFLVLFLGLVSLSICEEEKRENEDEEEQEDDEQSEMKRGLWSKIKEV GKEAAKAAAKAAGKAALGAVSEAVGEQ (SEQ ID NO: 2) (Amiche et al. (1994) J. Biol. Chem. 269:17847-17852). L'ADNc correspondant à ce précurseur a été identifié et est montré dans la séquence SEQ ID NO: 3.

La dermaseptine B2 est connue pour augmenter la liaison des agonistes au récepteur de l'adénosine A1 (Daly et al. (1992) Proc. Natl. Acad. Sci. USA 89:10960-10963; Moni et al. (1995) Cell. Mol. Neurobiol. 15:465-493). Une analyse structurale et pharmacologique a montré qu'elle s'apparentait à la grande famille des dermaseptines, peptides antimicrobiens à large spectre, isolés à partir de rainettes amazoniennes (Amiche et al. (1994) J. Biol. Chem. 269:17847-17852; Charpentier et al. (1998) J. Biol. Chem. 273:14690-14697; Mor et al. (1994) J. Biol. Chem. 269:31635-31641; Mor et al. (1991) Biochemistry 30:8824-8830). La synthèse chimique en phase solide de ce peptide n'offre aucune difficulté particulière car il est de taille relativement courte et sa structure n'est pas ou peu altérée par des modifications post-traductionnelles. De plus, sa production par expression recombinante chez *Escherichia coli est* également possible du fait de la disponibilité de son ADNc.

Comme il est bien connu de l'homme du métier, la dermaseptine B2 tue rapidement à des doses micromolaires les bactéries Gram+ et Gram-, les levures, les protozoaires et les champignons filamenteux. Elle est de plus dépourvue d'activité hémolytique.

### Peptides

Les peptides selon l'invention présentent une activité biologique. Par "activité biologique", on entend ici notamment une activité inhibitrice de l'angiogenèse et/ou de la prolifération de cellules et/ou de la croissance de tumeurs. Un peptide selon l'invention présente une activité biologique dès lors qu'il présente au moins l'une des activités mentionnées ci-dessus. De préférence, les peptides présentent une activité inhibitrice de la prolifération de cellules et/ou de la croissance de cellules, en particulier des cellules tumorales ou vasculaires.

L'activité inhibitrice de la prolifération de cellules et/ou de la croissance des tumeurs d'un peptide peut aisément être évaluée *in vitro* ou *in vivo,* par l'homme du métier, notamment au moyen des tests suivants :
- *in vitro,* par (i) mise en contact des peptides avec des cellules de type fibroblastique (*e.g*. NIH 3T3) stimulées par un facteur de croissance, en l'absence de sérum, (ii) ajout de thymidine marquée par radioactivité, et (iii) mesure de la radioactivité incorporée par les cellules, par exemple par scintillation liquide ;
- *in vitro,* par mise en contact des peptides avec des cellules tumorales (*e.g*. PC-3) en agar mou, et observation de la croissance des cellules par mesure de leur diamètre ;
- *in vivo,* par injection des peptides à une souris nude dans laquelle des tumeurs ont été induites par injection de PC-3, et observation de la croissance des tumeurs par mesure du volume et/ou du poids des tumeurs.

Par peptide "isolé", on entend ici un peptide isolé de l'organisme d'un animal ou d'un microorganisme. Cependant, le peptide isolé peut par exemple être présent dans une composition pharmaceutique ou dans un kit. De manière préférée, le peptide est présent dans une des compositions pharmaceutiques décrites ci-dessous. Ce peptide est de préférence sous forme purifiée. Le peptide selon l'invention peut être synthétisé par voie chimique ou biologique. Il peut en particulier être produit de manière recombinante.

De manière préférée, le peptide selon l'invention a une taille comprise entre 6 et 81, 6 et 76, 6 et 71, 6 et 66, 6 et 61, 6 et 56, 6 et 51, 6 et 46, 6 et 41, 6 et 36, 6 et 33, 6 et 30, 6 et 28, 6 et 26, 6 et 24, 6 et 22, 6 et 20, 6 et 18, 6 et 16, 6 et 14, 6 et 12, 6 et 10 acides aminés, de préférence une taille de 9, 8 ou 7 acides aminés. De manière préférée entre toutes, le peptide selon l'invention a une taille de 33 acides aminés.

Ledit peptide peut en particulier comprendre ou consister en une séquence d'acides aminés choisie dans le groupe constitué des séquences SEQ ID NO: 1 et SEQ ID NO: 2; une séquence d'acides aminés ayant au moins 80% d'identité avec les séquences SEQ ID NO: 1 ou SEQ ID NO: 2; et un fragment de ces séquences, sous réserve que le peptide isolé inhibe la croissance et/ou la prolifération cellulaire. De manière préférée, ledit peptide consiste en une séquence d'acides aminés choisie dans le groupe constitué des séquences SEQ ID NO: 1 et SEQ ID NO: 2.

Ledit peptide peut également consister en un fragment de la séquence SEQ ID NO: 1 ou SEQ ID NO : 2.

Par "fragment" d'une séquence de référence, on entend ici une séquence constituée par un enchaînement d'acides aminés consécutifs d'une séquence de référence, et qui a une taille inférieure à la séquence de référence. Dans le cadre de cette invention, les fragments peuvent par exemple avoir une taille comprise entre 6 et 76, 6 et 71, 6 et 66, 6 et 61, 6 et 56, 6 et 51, 6 et 46, 6 et 41, 6 et 36, 6 et 33, 6 et 30, 6 et 28, 6 et 26, 6 et 24, 6 et 22, 6 et 20, 6 et 18, 6 et 16, 6 et 14, 6 et 12, 6 et 10 acides aminés, de préférence une taille de 9, 8 ou 7 acides aminés. En particulier, les fragments peuvent avoir une taille de 33 acides aminés. De préférence, ces fragments sont issus de la partie C-terminale de la dermaseptine B2 ou du précurseur de la dermaseptine B2.

Les peptides selon l'invention incluent en outre également des peptides ayant des séquences dérivées de la séquence SEQ ID NO: 1 ou SEQ ID NO: 2, ou dérivées de fragments de la séquence SEQ ID NO: 1 ou SEQ ID NO: 2, définies par un pourcentage d'identité de séquence par rapport à l'une des séquences SEQ ID NO: 1 ou SEQ ID NO: 2. Ces séquences dérivées peuvent différer de la séquence de référence par substitution, délétion et/ou insertion d'un ou de plusieurs acides aminés, et ceci à des positions telles que ces modifications ne portent pas significativement atteinte à l'activité biologique des peptides. Les substitutions peuvent notamment correspondre à des substitutions conservatives ou à des substitutions d'acides aminés naturels par des acides aminés non naturels ou pseudo-acides aminés.

Par "séquence d'acides aminés au moins 80% (par exemple) identique à une séquence de référence", on entend une séquence identique à la séquence de référence si ce n'est que cette séquence peut comporter jusqu'à vingt mutations (substitutions, délétions et/ou insertions) pour chaque partie de cent acides aminés de la séquence de référence. Ainsi, pour une séquence de référence faisant 100 acides aminés, un fragment de 80 acides aminés et une séquence de 100 acides aminés comportant 20 substitutions par rapport à la séquence de référence sont deux exemples de séquences 80% identiques à la séquence de référence.

Le pourcentage d'identité est généralement déterminé en utilisant un logiciel d'analyse de séquence (par exemple, le Sequence Analysis Software Package of the Genetics Computer Group, University of Wisconsin Biotechnology Center, 1710 University Avenue, Madison, WI 53705). Les séquences d'acides aminés à comparer sont alignées pour obtenir le maximum de degré d'identité. A cette fin, il peut être nécessaire d'introduire de manière artificielle des espaces ("gaps") dans la séquence. L'alignement peut être réalisé manuellement ou automatiquement. Des algorithmes d'alignement automatisé de séquences nucléotidiques sont bien connus de l'homme du métier et sont par exemple décrits dans Altschul et al. (1997) Nucleic Acids Res. 25:3389 et mis en oeuvre par des logiciels tels que le logiciel Blast. Un algorithme pouvant être isolé est par exemple l'algorithme de Needleman-Wunsch (Needleman et Wunsch (1970) J Mol Biol. 48:443-53). Une fois l'alignement optimal réalisé, le degré d'identité est établi par enregistrement de toutes les positions pour lesquelles les acides aminés des deux séquences comparées sont identiques, par rapport au nombre total de positions.

Ainsi, les peptides selon l'invention peuvent comprendre ou consister en une séquence choisie parmi :
- un fragment d'une séquence au moins 80%, 85%, 90%, 95% ou 100% identique à la séquence SEQ ID NO: 1 ou SEQ ID NO : 2; et
- une séquence au moins 80%, 85%, 90%, 95% ou 100% identique à la séquence SEQ ID NO: 1 ou SEQ ID NO : 2.

Dans un mode de réalisation particulier, la séquence des peptides diffère de la séquence SEQ ID NO: 1 ou SEQ ID NO: 2, ou d'un fragment de la séquence SEQ ID NO: 1 ou SEQ ID NO: 2, uniquement par la présence de substitutions conservatives. Les substitutions conservatives sont des substitutions d'acides aminés de même classe, telles que des substitutions d'acides aminés aux chaînes latérales non chargées (tels que l'asparagine, la glutamine, la sérine, la cystéine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique), d'acides aminés aux chaînes latérales apolaires (tels que l'alanine, la valine, la leucine, l'isoleucine, la proline, la phénylalanine, la méthionine et le tryptophane).

Conformément à l'invention, les peptides peuvent être modifiés chimiquement ou enzymatiquement pour améliorer leur stabilité ou leur biodisponibilité. De telles modifications chimiques ou enzymatiques sont bien connues de l'homme de l'art. De manière non limitative, on peut par exemple citer les modifications suivantes:
- modifications de l'extrémité C-terminale ou N-terminale des peptides tels que la désamination ou l'acylation N-terminale (de préférence l'acétylation), ou tels que l'amidation ou l'estérification C-terminale;
- modifications de la liaison amide entre deux acides aminés, tels que l'acylation (de préférence l'acétylation) ou l'alkylation au niveau de l'azote ou du carbone alpha ;
- changements de chiralité, tels que la substitution d'un acide aminé naturel (L-énanthiomère) par le D-énanthiomère correspondant. Cette modification peut éventuellement être accompagnée par une inversion de la chaîne latérale (de l'extrémité C-terminale à l'extrémité N-terminale) ;
- changements en azapeptides, dans lesquels un ou plusieurs carbones alpha sont remplacés par des atomes azote ; et/ou
- changements en betapeptides, dans lesquels on ajoute un ou plusieurs carbones du coté N-alpha ou du côté C-alpha de la chaîne principale.

A ce titre, on peut modifier un ou plusieurs des acides aminés lysine (K) des peptides, notamment par :
- amidation : cette modification est simple à accomplir, la charge positive de la lysine étant substituée par des groupements hydrophobes (par exemple, acétyle ou phénylacétyle) ;
- amination : par formation d'amides secondaires à partir de l'amine primaire R = (CH₂)₄-NH₃⁺, par exemple en formant des groupements N-méthyle, N-allyle ou N-benzyle ; et
- par formation des groupements N-oxyde, N-nitroso, N-dialkyl phosphoryle, N-sulfényle, ou N-glycoside.

On peut par ailleurs aussi ou alternativement modifier un ou plusieurs acides aminés thréonine (T) et/ou sérine (S) des peptides, notamment en introduisant au niveau du groupe OH de la chaîne latérale de la thréonine et/ou de la sérine, un groupement ester ou éther. L'estérification, opération simple, peut être réalisée à l'aide d'un acide carboxylique, d'un anhydride, par pontages, etc, pour former par exemple des acétates ou des benzoates. L'éthérification, qui donne des composés plus stables, peut être effectuée à l'aide d'un alcool, d'un halogénure, etc, pour former par exemple un méthyl éther ou un O-glycoside.

On peut par ailleurs aussi ou alternativement modifier un ou plusieurs acides aminés glutamine (Q) par exemple par amidation, en formant des amines secondaires ou tertiaires, notamment avec des groupements de type méthyl, éthyl, fonctionnalisés ou non.

On peut par ailleurs aussi ou alternativement modifier un ou plusieurs acides aminés glutamate (E) et/ou aspartate (D), par exemple :
- par estérification, pour former des méthyl esters substitués ou non, des éthyl esters, benzyl esters, des thiols (esters activés) ; et
- par amidation, notamment pour former des groupements N,N diméthyle, nitroanilides, pyrrolidinyles.

En revanche, il est préférable de ne pas modifier les acides aminés proline, qui participent à la structure secondaire des peptides, sachant en outre que les acides aminés G, A et M n'offrent généralement pas de possibilités de modifications qui seraient manifestement intéressantes.

### Molécule chimère

La description décrit également une molécule chimère comprenant au moins un peptide selon l'invention tel que défini ci-dessus, dans laquelle ledit peptide est lié à :
a) un composé thérapeutique utile pour le traitement de désordres prolifératifs;
b) une enzyme capable de convertir une molécule en un composé thérapeutique utile pour le traitement de désordres prolifératifs; ou
c) une molécule porteuse.

Par "molécule chimère", on entend ici une molécule comprenant ou consistant en un peptide selon l'invention lié à une autre molécule. Le peptide selon l'invention est lié à l'autre molécule par une liaison covalente. La liaison correspond de préférence à un couplage chimique. Cependant, lorsque la molécule auquel le peptide est lié est un autre polypeptide, les deux peptides peuvent se présenter sous la forme d'une protéine de fusion.

Par "molécule porteuse", on entend ici toute molécule à laquelle au moins un peptide peut être couplé (conjugué). De préférence, la molécule porteuse possède une taille suffisante pour permettre de lui coupler au moins 3 peptides selon l'invention, de préférence de 3 à 8 peptides selon l'invention. Au sens de l'invention, on entend par "couplés", pour un peptide, le fait d'être lié à la molécule porteuse par une liaison covalente, soit directement, soit par l'intermédiaire d'un composé espaceur entre le peptide et la molécule porteuse. Des exemples d'espaceurs acceptables comprennent des composés de type éthylène glycol, pipérazine, ou un acide aminé de type acide aminohexanoïque ou beta-alanine.

L'homme de l'art connaît de telles molécules porteuses à laquelle un peptide peut avantageusement être couplé.

Par exemple, des peptides sont couramment couplés à la Keyhole Limpet Hemocyanin (KLH), la sérum albumine bovine (BSA), l'ovalbumine (OVA), la thyroglobuline (THY) ou la MAP (multiple antigenic peptide).

Dans un mode de réalisation préféré, la molécule porteuse correspond à un support tel que décrit dans la demande PCT publiée sous le numéro WO 2007/125210. Un tel support peut notamment être choisi parmi un peptide linéaire ou un peptide cyclique, un peptoïde (oligomère de glycine N-substitué) linéaire ou cyclique, un foldamère (oligomère ou polymère ayant une forte tendance à adopter une conformation compacte bien définie et prévisible en solution), un polymère linéaire ou un dendrimère (macromolécule constituée de monomères qui s'associent selon un processus arborescent autour d'un coeur central plurifonctionnel) sphérique, un sucre, ou une nanoparticule. Avantageusement, ledit support est choisi parmi un peptide linéaire ou cyclique, ou bien un peptoïde linéaire ou cyclique. L'utilisation d'un peptide linéaire permet une synthèse facile du support. Un peptide linéaire servant de support dans l'invention peut avantageusement comprendre une proportion de lysine supérieure à 25%. Plus précisément, lorsqu'un peptide linéaire est utilisé comme support dans l'invention, le(s) peptide(s) selon l'invention sont de préférence greffés sur une lysine. Dans le cas où le support est un peptide linéaire ou cyclique et où le(s) peptide(s) selon l'invention sont greffés directement sur le peptide, la liaison entre le peptide support et le(s) peptide(s) selon l'invention est de préférence réalisée au niveau d'un résidu lysine du peptide support, au niveau d'un groupe amino en position α ou ε, de préférence au niveau du groupe amino en position ε (sur la chaîne latérale) de la lysine. Ainsi, le greffage direct du(des) peptide(s) selon l'invention sur un support peptidique se fait avantageusement par une liaison amide entre une fonction acide COOH de l'acide aminé en position C-terminale du motif pseudopeptidique et un groupement amino d'un résidu lysine, de préférence le groupement amino en position ε (sur la chaîne latérale) de la lysine. Avantageusement, le support d'un peptide selon l'invention est choisi parmi un hexapeptide cyclique constitué en alternance de résidus alanine (A) de configuration D et de résidus lysine (K) de configuration L.

La molécule chimère selon l'invention peut également comprendre au moins un peptide selon l'invention lié à une enzyme ou à un composé thérapeutique utile pour le traitement de désordres prolifératifs.

Dans un mode de réalisation préféré, le peptide selon l'invention est lié à un composé cytotoxique.

De manière alternative, le peptide selon l'invention peut être lié à une enzyme capable de convertir une pro-drogue en un composé thérapeutique utile pour le traitement de désordres prolifératifs (voir par exemple les brevets US 5 760 072 et US 5 433 955).

Le peptide selon l'invention peut par exemple être lié à une enzyme présente dans l'environnement matriciel telle que les membres de la famille des métalloprotéinases matricielles, l'urokinase ou la plasmine.

Le peptide selon l'invention peut par exemple être lié à un composé thérapeutique sélectionné parmi le groupe constitué par un segment N-terminal de l'annexine 1 humaine, les cytokines anti-inflammatoires (en particulier l'IL10 et l'IL13), les inhibiteurs non activants des récepteurs membranaires des cytokines pro-inflammatoires, les glucocorticoïdes, les anti-inflammatoires non stéroïdiens, et le méthotrexate.

Le peptide selon l'invention peut être lié au composé thérapeutique par le biais d'un segment de liaison qui est reconnu et clivé par une enzyme ou un ensemble d'enzymes spécifiques de l'environnement des cellules cancéreuses. Plus particulièrement, cette enzyme peut être choisie parmi une métalloprotéase de la matrice extracellulaire, une urokinase, et une protéase spécifique du clivage du segment extracellulaire des cytokines membranaires ou de leurs récepteurs. Le clivage du segment de liaison au niveau des cellules cancéreuses permet alors de libérer deux principes actifs : le peptide selon l'invention d'une part, et le composé thérapeutique d'autre part.

De telles molécules chimères peuvent être utilisées en tant que médicament, plus particulièrement pour le traitement ou la prévention d'un désordre prolifératif, d'une lésion oculaire et/ou d'une maladie autoimmune. De préférence, de telles molécules chimères sont utilisées pour le traitement ou la prévention d'un désordre prolifératif comme le cancer.

### Utilisation thérapeutique

Les peptides de l'invention présentent des propriétés d'inhibition de la prolifération de cellules et/ou de la croissance de cellules. A ce titre ces peptides et les molécules chimères les comprenant sont particulièrement utiles pour le traitement de diverses pathologies associées à la prolifération et à la croissance de cellules.

La présente description décrit donc un peptide ou une molécule chimère selon l'invention pour leur utilisation dans le traitement ou la prévention d'un désordre prolifératif, d'une lésion oculaire et/ou d'une maladie auto-immune, en particulier pour inhiber la croissance et/ou la prolifération cellulaire.

Par "désordre prolifératif", on entend ici toute prolifération anormale de cellules, qu'elle soit bénigne ou maligne (cancéreuse). Les peptides de l'invention sont tout particulièrement utiles dans le cadre du traitement et/ou de la prévention de cancers.

Les désordres prolifératifs incluent notamment les tumeurs. L'invention vise tout particulièrement les tumeurs cancéreuses, qu'elles soient solides ou non. L'invention vise le traitement et/ou la prévention de tumeurs solides telles que les mélanomes, les carcinomes, les sarcomes, le rhabdomyosarcome, le rétinoblastome, le neuroblastome, l'ostéosarcome, le glioblastome, les tumeurs (primitives ou non) mammaires, ovariennes, pulmonaires, du col de l'utérus, du tractus digestif, notamment du colon, du système urologique, du foie, du pancréas, des os. Les tumeurs non solides sont également visées, à savoir notamment les leucémies ou les lymphomes. S'agissant toujours des tumeurs cancéreuses, les peptides selon l'invention sont particulièrement utiles pour le traitement des métastases tumorales, et/ou pour la prévention de leur formation. Parmi les tumeurs bénignes, également visées par la présente invention, on peut citer les hémangiomes et les adénomes hépatocellulaires.

Les désordres prolifératifs incluent également des désordres autres que les tumeurs, tels que la polyarthrite rhumatoïde (RA), qui est une maladie inflammatoire associée à une intense angiogenèse, ainsi que des maladies de peau comme le psoriasis. Dans cette pathologie on observe une prolifération des synoviocytes, qui est à la base de la création d'un pannus inflammatoire. Les mécanismes associés à ce type de pathologie ressemblent aux mécanismes menant à une croissance tumorale.

Les "lésions oculaires" incluent notamment des pathologies de la rétine telles que la rétinopathie diabétique, la dégénérescence maculaire, l'occlusion de la veine ou de l'artère rénale, le glaucome. Les peptides peuvent également être utiles pour traiter des lésions oculaires pouvant être la conséquence d'actes de chirurgie réparatrice comme la greffe de cornée. Il existe notamment une forme de dégénérescence maculaire liée à l'âge dite "exsudative". Ce type de pathologie entraîne la formation de vaisseaux sanguins anormaux sous la rétine. Cette augmentation anarchique de vaisseaux peut à long terme endommager la macula et conduire à la cécité. Concernant la rétinopathie diabétique, cette pathologie est une maladie des capillaires rétiniens qui deviennent anormaux avec notamment une disparition des péricytes. Une rupture de la barrière hemato-rétinienne est ensuite observée, entraînant une hyperperméabilité vasculaire. Ces altérations vont entraîner des ischémies rétiniennes, qui vont être à la base d'une néoangiogenése menant à plus long terme à la cécité. Dans ces types de pathologie l'étiopathologie repose sur le développement d'un réseau vasculaire anarchique. Ainsi, l'utilisation de molécules anti-angiogéniques comme les peptides selon l'invention est un traitement thérapeutique efficace contre ces pathologies.

Les peptides décrits ici possédant des propriétés anti- angiogéniques, ils sont utiles pour traiter ou prévenir des maladies autoimmunes (Griffioen et al. (1999) Int J Cancer. 80:315-9; Griffioen (2008) Cancer Immunol Immunother. 57:1553-8). Les "maladies autoimmunes" incluent notamment la sclérose en plaque (MS), les maladies inflammatoires de l'intestin (IBD) notamment la maladie de Crohn, et le lupus érythémateux.

Enfin, les peptides et molécules chimères décrits ici peuvent également être utiles comme composés abortifs, pour le contrôle des naissances, en bloquant l'angiogenèse utérine et donc l'implantation de l'embryon.

Les désordres prolifératifs peuvent être traités à tout stade de la prolifération. Par "traitement", on entend traitement à titre curatif (visant à au moins soulager, freiner ou stopper le développement de la pathologie). Par "prévention", on entend traitement à titre prophylactique (visant à réduire le risque d'apparition de la pathologie).

Les peptides et molécules chimères selon l'invention peuvent également être utilisés en combinaison avec un second principe actif visant au traitement ou à la prévention de la même maladie. Dans le cadre du traitement et/ou de la prévention de cancers, les peptides peuvent par exemple être utilisés en combinaison avec de la chirurgie visant à enlever les tumeurs, une radiothérapie, une chimiothérapie, une hormonothérapie, et/ou une immunothérapie.

La description décrit donc également un peptide ou une molécule chimère selon l'invention, en combinaison avec au moins un composé thérapeutique utile pour le traitement de désordres prolifératifs, de lésions oculaires ou de maladies auto-immunes, pour leur utilisation dans le traitement et/ou la prévention d'un désordre prolifératif, d'une lésion oculaire ou d'une maladie auto-immune.

Par "composé thérapeutique utile pour le traitement de désordres prolifératifs, de lésions oculaires ou de maladies auto-immunes", on entend tout principe actif autre que les peptides selon l'invention qui est utile pour le traitement et/ou la prévention de désordres prolifératifs, de lésions oculaires ou de maladies auto-immunes. Il est par exemple possible de combiner les peptides selon l'invention avec un principe actif disposant déjà d'une autorisation de mise sur le marché visant au traitement de ces maladies. De tels composés thérapeutiques utiles pour le traitement de désordres prolifératifs incluent notamment les composés suivants, déjà approuvés pour le traitement de divers cancers : Abraxane, Adriamycin (Doxorubicin Hydrochloride), Adrucil (Fluorouracil), Aldara (Imiquimod), Alemtuzumab, Alimta, Pemetrexed Disodium), Aminolevulinic Acid, Anastrozole, Aprepitant, Arimidex (Anastrozole), Aromasin (Exemestane), Arranon (Nelarabine), Arsenic Trioxide, Avastin (Bevacizumab), Azacitidine, Bendamustine Hydrochloride, Bevacizumab, Bexarotene, Bexxar (Tositumomab and I 131 Iodine Tositumomab), Bortezomib, Campath (Alemtuzumab), Camptosar (Irinotecan Hydrochloride), Capecitabine, Carboplatin, Cetuximab, Cisplatin, Clafen (Cyclophosphamide), Clofarabine, Clofarex (Clofarabine), Clolar (Clofarabine), Cyclophosphamide, Cytarabine, Cytosar-U (Cytarabine), Cytoxan (Cyclophosphamide), Dacogen (Decitabine), Dasatinib, Decitabine, DepoCyt (Liposomal Cytarabine), DepoFoam (Liposomal Cytarabine), Dexrazoxane Hydrochloride, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), Efudex (Fluorouracil), Ellence (Epirubicin Hydrochloride), Eloxatin (Oxaliplatin), Emend (Aprepitant), Epirubicin Hydrochloride, Erbitux (Cetuximab), Erlotinib Hydrochloride, Evacet (Doxorubicin Hydrochloride Liposome), Evista (Raloxifene Hydrochloride), Exemestane, Faslodex (Fulvestrant), Femara (Letrozole), Fluoroplex (Fluorouracil), Fluorouracil, Fulvestrant, Gefitinib, Gemcitabine Hydrochloride, Gemtuzumab Ozogamicin, Gemzar (Gemcitabine Hydrochloride), Gleevec (Imatinib Mesylate), Herceptin (Trastuzumab), Hycamtin (Topotecan Hydrochloride), Ibritumomab Tiuxetan, Imatinib Mesylate, Imiquimod, Iressa (Gefitinib), Irinotecan Hydrochloride, Ixabepilone, Ixempra (Ixabepilone), Keoxifene (Raloxifene Hydrochloride), Kepivance (Palifermin), Lapatinib Ditosylate, Lenalidomide, Letrozole, Levulan (Aminolevulinic Acid), LipoDox (Doxorubicin Hydrochloride Liposome), Liposomal Cytarabine, Methazolastone (Temozolomide), Mylosar (Azacitidine), Mylotarg (Gemtuzumab Ozogamicin), Nanoparticle Paclitaxel (Paclitaxel Albumin-stabilized Nanoparticle Formulation), Nelarabine, Neosar (Cyclophosphamide), Nexavar (Sorafenib Tosylate), Nilotinib, Nolvadex (Tamoxifen Citrate), Oncaspar (Pegaspargase), Oxaliplatin, Paclitaxel, Paclitaxel Albumin-stabilized Nanoparticle Formulation, Palifermin, Panitumumab, Paraplat (Carboplatin), Paraplatin (Carboplatin), Pegaspargase, Pemetrexed Disodium, Platinol-AQ (Cisplatin), Platinol (Cisplatin), Raloxifene Hydrochloride, Revlimid (Lenalidomide), Rituxan (Rituximab), Rituximab, Sclerosol Intrapleural Aerosol (Talc), Sorafenib Tosylate, Sprycel (Dasatinib), Sterile Talc Powder (Talc), Steritalc (Talc), Sunitinib Malate, Sutent (Sunitinib Malate), Synovir (Thalidomide), Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Temodar (Temozolomide), Temozolomide, Temsirolimus, Thalomid (Thalidomide), Thalidomide, Totect (Dexrazoxane Hydrochloride), Topotecan Hydrochloride, Torisel (Temsirolimus), Tositumomab and l 131 lodine Tositumomab, Trastuzumab, Treanda (Bendamustine Hydrochloride), Trisenox (Arsenic Trioxide), Tykerb (Lapatinib Ditosylate), Vectibix (Panitumumab), Velcade (Bortezomib), Vidaza (Azacitidine), Vorinostat, Xeloda (Capecitabine), Zevalin (Ibritumomab Tiuxetan), Zinecard (Dexrazoxane Hydrochloride), Zoledronic Acid, Zolinza (Vorinostat) et Zometa (Zoledronic Acid).

La description décrit en outre une méthode pour traiter ou prévenir un désordre prolifératif ou une lésion oculaire chez un mammifère, plus particulièrement un humain, comprenant l'administration d'une quantité thérapeutiquement efficace d'au moins un peptide tel que décrit ci-dessus ou d'au moins une molécule chimère tel que décrit ci-dessus, éventuellement en combinaison avec au moins un composé thérapeutique utile pour le traitement de désordres prolifératifs, de lésions oculaires ou de maladies auto-immunes. Ledit mammifère est préférentiellement un mammifère souffrant ou susceptible de souffrir d'un désordre prolifératif, d'une lésion oculaire et/ou d'une maladie auto-immune.

La description décrit aussi une composition thérapeutique comprenant un peptide ou une molécule chimère tels que décrits ci-dessus et, éventuellement un ou plusieurs excipients pharmaceutiquement acceptables.

### Acides nucléiques et vecteurs

Dans un mode de réalisation préféré, l'invention concerne l'utilisation ou l'administration d'un peptide ou d'une molécule chimère selon l'invention.

Cependant, on peut également choisir d'employer une thérapie génique, en utilisant ou en administrant un acide nucléique codant pour un peptide selon l'invention à la place du peptide. Il s'agit alors d'administrer à un patient un acide nucléique qui code pour le ou les peptides d'intérêt, dans des conditions telles que le ou les peptides sont exprimés *in vivo* par les cellules du patient dans lesquelles l'acide nucléique a été transféré.

La description décrit donc aussi des acides nucléiques comprenant ou consistant en une séquence codant un peptide selon l'invention. De tels acides nucléiques peuvent aisément être obtenus par clonage de fragments de l'ADNc codant la dermaseptine B2 ou le précurseur de la dermaseptine B2. Plus particulièrement, un acide nucléique comprenant ou codant un peptide selon l'invention est représenté par la séquence SEQ ID NO: 3.

Un tel acide nucléique codant un peptide selon l'invention peut être notamment sous la forme d'un vecteur d'ADN, par exemple un vecteur plasmidique. On peut administrer un ou plusieurs vecteurs, chaque vecteur pouvant porter une ou plusieurs séquence(s) codant pour au moins un des peptides selon l'invention. Dans ce vecteur, la ou les séquence(s) codant pour au moins un des peptides selon l'invention sont liées de manière fonctionnelle à un ou des élément(s) permettant son expression ou la régulation de son expression, tels que notamment des promoteurs, activateurs et/ou terminateurs de transcription.

Selon un mode de réalisation préféré, on utilise un vecteur portant une séquence codant le peptide de séquence SEQ ID NO: 1 ou SEQ ID NO: 2. Selon un mode de réalisation davantage préféré, on utilise un vecteur portant un acide nucléique de séquence SEQ ID NO: 3.

Le ou les vecteurs d'ADN peuvent être introduits *in vivo* par toute technique connue de l'homme du métier. En particulier, il est possible d'introduire le vecteur d'ADN *in vivo* sous une forme nue, c'est-à-dire sans l'aide d'un quelconque véhicule ou système qui faciliterait la transfection du vecteur dans les cellules (EP 465 529).

Un canon à gènes peut être également employé, par exemple en déposant l'ADN à la surface de particules "en or" et en projetant celles-ci de manière à ce que l'ADN pénètre à travers la peau d'un patient (Tang et al., (1992) Nature 356:152-4). Des injections au moyen d'un gel liquide sont également possibles pour transfecter à la fois peau, muscle, tissu gras et tissu mammaire (Furth et al., (1992) Anal Biochem. 205:365-8).

Des techniques de microinjection, électroporation, précipitation au phosphate de calcium, formulations à l'aide de nanocapsules ou de liposomes sont d'autres techniques disponibles.

Des nanoparticules en polyalkyl cyanoacrylate biodégradables sont particulièrement avantageuses. Dans le cas de liposomes, l'utilisation de lipides cationiques favorise l'encapsulation des acides nucléiques qui sont chargés négativement, et facilite la fusion avec les membranes cellulaires chargées négativement.

De manière alternative, le vecteur peut être sous la forme d'un virus recombinant comprenant, insérée dans son génome, une séquence d'acide nucléique qui code pour le ou lesdits peptide(s).

Le vecteur viral peut être de préférence choisi parmi un adénovirus, un rétrovirus, en particulier un lentivirus, ainsi qu'un virus adéno-associé (AAV), un virus de l'herpès, un cytomégalovirus (CMV), un virus de la vaccine, etc. Des vecteurs lentivirus ont par exemple été décrits par Firat et al., (2002) J Gene Med 4:38-45.

De manière avantageuse, le virus recombinant est un virus défectif. Le terme "virus défectif" désigne un virus incapable de se répliquer dans une cellule cible. Généralement, le génome des virus défectifs est dépourvu d'au moins les séquences nécessaires pour la réplication dudit virus dans la cellule infectée. Ces régions peuvent être soit éliminées, soit rendues non fonctionnelles ou encore substituées par d'autres séquences et en particulier par l'acide nucléique qui code pour le peptide d'intérêt. Néanmoins, de préférence, le virus défectif conserve les séquences de son génome qui sont nécessaires pour l'encapsulation des particules virales.

Une administration ciblée de gènes est par exemple décrite dans la demande WO 95/28 494.

### Production des peptides et molécules chimères

Les polypeptides utiles dans la présente invention peuvent être synthétisés par n'importe quelle méthode bien connue de l'homme du métier. De telles méthodes incluent notamment la synthèse chimique classique (en phase solide ou en phase homogène liquide), la synthèse enzymatique à partir d'acides aminés constitutifs ou de leurs dérivés, ainsi que des procédés de production biologiques par des cellules hôtes recombinantes.

La synthèse par voie chimique est particulièrement avantageuse pour des raisons de pureté, de spécificité antigénique, d'absence de produits secondaires non désirés et pour sa facilité de production. Le peptide obtenu peut ensuite éventuellement être purifié selon n'importe quelle méthode bien connue de l'homme de l'art. Le procédé de production peut également comprendre une ou des étapes de modification chimique ou enzymatique du peptide pour améliorer sa stabilité ou sa biodisponibilité, ainsi qu'une ou des étapes pour lier le peptide à un composé thérapeutique.

La synthèse par voie chimique inclut, entre autres méthodes, la synthèse de type Merrifield et la méthodologie de synthèse peptidique en phase solide Fmoc (voir par exemple "Fmoc solid Phase peptide synthesis, a practical approach", publié par W.C. Chan et P. D. White, Oxford university press, 2000).

La présente description décrit en outre un procédé de production d'une molécule chimère selon l'invention, comprenant:
a) la synthèse d'un peptide selon l'invention, de préférence par voie chimique,
b) la conjugaison dudit peptide avec un composé thérapeutique utile pour le traitement de désordres prolifératifs, une enzyme capable de convertir une molécule en un composé thérapeutique utile pour le traitement de désordres prolifératifs, ou une protéine porteuse.

Le procédé de production de la molécule chimère décrit ici peut en outre comprendre une étape de formulation de la molécule chimère obtenu en une composition pharmaceutique, par exemple l'une des compositions décrites dans le paragraphe ci-dessous.

Le peptide selon l'invention peut également être obtenu par un procédé de production biologique par une cellule hôte recombinante. Dans un tel procédé, un vecteur contenant un acide nucléique codant pour un peptide selon l'invention est transféré dans une cellule hôte qui est mise en culture dans des conditions permettant l'expression du peptide correspondant.

Le peptide produit peut ensuite être récupéré et purifié.

Les procédés de purification utilisés sont connus de l'homme du métier. Le peptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.

La séquence d'acide nucléique d'intérêt peut être insérée dans un vecteur d'expression, dans lequel elle est liée de manière fonctionnelle à un ou des élément(s) permettant son expression ou la régulation de son expression, tels que notamment des promoteurs, activateurs et/ou terminateurs de transcription.

Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression tels que décrits ci-dessus, contenant une séquence nucléotidique définie selon l'invention sont également décrits ici.

La description décrit en outre les cellules hôtes transfectées, de manière transitoire ou stable, par ces vecteurs d'expression. Ces cellules peuvent être obtenues par l'introduction dans des cellules hôtes, procaryotes ou eucaryotes, d'une séquence nucléotidique insérée dans un vecteur tel que défini ci-dessus, puis la mise en culture desdites cellules dans des conditions permettant la réplication et/ou l'expression de la séquence nucléotidique transfectée.

Des exemples de cellules hôtes incluent notamment des cellules humaines telles que HEK293, PER.C6, des cellules de mammifères non humains telles que CHO, COS, MDCK, des cellules d'insectes telles que les cellules SF9, des bactéries telles que *Escherichia coli,* des souches de champignons et/ou de levures telles que L40 et Y90.

### Compositions pharmaceutiques

La présente description décrit également une composition pharmaceutique comprenant, en tant que principe actif, au moins un peptide ou une molécule chimère selon l'invention. Généralement de telles compositions comprennent un ou plusieurs excipients pharmaceutiquement acceptables.

Le peptide et la molécule chimère selon l'invention peuvent correspondre à n'importe lequel des peptides et molécules chimères décrits ci-dessus. Selon un mode de réalisation préféré de l'invention, le peptide est un peptide de séquence SEQ ID NO: 1 ou SEQ ID NO: 2.

Par "excipient" ou "véhicule pharmaceutiquement acceptable", on entend tout solvant, milieu de dispersion, agents retardant l'absorption etc, qui ne produisent pas de réaction secondaire, par exemple allergique, chez l'humain ou l'animal.

De manière alternative, la description décrit également une composition pharmaceutique comprenant, en tant que principe actif, un acide nucléique codant un peptide selon l'invention, de préférence lié de manière fonctionnelle à un ou des élément(s) permettant l'expression du peptide ou la régulation de son expression, avec un ou plusieurs excipients pharmaceutiquement acceptables. Une composition pharmaceutique préférée comprend un acide nucléique codant un peptide de séquence SEQ ID NO : 1 ou SEQ ID NO: 2.

La description décrit également une composition pharmaceutique comprenant au moins un peptide, une molécule chimère ou un acide nucléique selon l'invention et au moins un composé thérapeutique utile pour le traitement de désordres prolifératifs, de lésions oculaires et/ou de maladies auto-immunes, en présence d'un ou plusieurs excipients pharmaceutiquement acceptables.

La description décrit également l'administration essentiellement simultanée de compositions séparées comprenant d'une part au moins un peptide, une molécule chimère ou un acide nucléique selon l'invention et d'autre part au moins un composé thérapeutique utile pour le traitement de désordres prolifératifs, de lésions oculaires et/ou de maladies auto-immunes.

L'administration peut également être réalisée de manière séquentielle, au moyen de compositions séparées comprenant d'une part au moins un peptide, une molécule chimère ou un acide nucléique selon l'invention et d'autre part au moins un composé thérapeutique utile pour le traitement de désordres prolifératifs, de lésions oculaires et/ou de maladies auto-immunes.

La posologie dépend naturellement de l'actif considéré, du mode d'administration, de l'indication thérapeutique, de l'âge du patient et de son état.

La dose de peptide est préférablement de 0,1 à 250 mg/kg par jour, de préférence de 1 à 100 ou de 0,5 à 100 mg/kg par jour, en particulier de 0,5 à 5 mg/kg. La dose unitaire du peptide comprend de préférence de 12,5 à 200 mg du peptide.

Quand les compositions pharmaceutiques comprennent des acides nucléiques, les doses d'acide nucléique (séquence ou vecteur) à administrer sont adaptées également en fonction notamment du mode d'administration, de la pathologie ciblée ainsi que de la durée de traitement. Généralement, lorsque des virus recombinants sont utilisés, ceux-ci sont formulés et administrés sous la forme de doses d'environ 10⁴ à 10¹⁴ pfu/ml, de préférence 10⁶ à 10¹⁰ pfu/ml. Le terme "pfu" (unité formant plage) correspond à l'infectivité d'une solution virale, et peut être déterminée en infectant une culture cellulaire appropriée et en mesurant, généralement après 48 heures, le nombre de plages de cellules infectées. Les techniques pour déterminer le titre pfu d'une solution virale sont bien décrites par la littérature.

Les compositions pharmaceutiques décrites ici peuvent être formulées de manière à être administrées au patient par une unique voie ou par des voies différentes.

Les compositions pharmaceutiques décrites ici peuvent par exemple être administrées par voie parentérale, en particulier par voie intraveineuse, sous-cutanée ou intramusculaire, par voie orale, par inhalation ou par application topique ou oculaire.

Lorsque l'administration par voie parentérale est envisagée, plus particulièrement par injection, les compositions décrites ici comprenant le ou les principes actifs se trouvent sous la forme de solutés et suspensions injectables conditionnées en ampoules ou flacons pour perfusion lente. L'injection peut notamment être réalisée par voie sous-cutanée, intramusculaire ou intraveineuse.

De manière préférée, en particulier dans le cas d'une tumeur solide, la composition pharmaceutique peut être injectée dans la tumeur.

Dans le cas d'une administration par voie orale, les compositions décrites ici se trouvent sous la forme de gélules, comprimés effervescents, comprimés nus ou enrobés, sachets, dragées, ampoules ou solutés buvables, microgranules ou formes à libération prolongée.

Les formes pour l'administration parentérale sont obtenues de façon conventionnelle par mélange du ou des principes actifs avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses.

A titre de tampon, l'homme du métier pourra utiliser des tampons à base de sels de phosphate organique.

Des exemples d'agents de mise en suspension englobent le méthylcellulose, l'hydroxyéthylcellulose, l'hydroxypropylcellulose, l'acacia et la carboxyméthylcellulose sodique.

En outre, des stabilisants utiles selon l'invention sont le sulfite de sodium et le métasulfite de sodium, tandis que l'on peut citer le p-hydroxybenzoate de sodium, l'acide sorbique, le crésol et le chlorocrésol en tant que conservateurs. Pour la préparation de solution ou de suspension orale, les principes actifs sont dissous ou mis en suspension dans un véhicule approprié avec un agent dispersant, un agent humectant, un agent de mise en suspension (par exemple la polyvinylpyrrolidone), un conservateur (tel que le méthylparaben ou le propylparaben), un agent correcteur de goût ou un colorant.

Pour la préparation de microcapsules, les principes actifs sont combinés à des diluants appropriés, des stabilisants appropriés, des agents favorisant la libération prolongée des substances actives ou tout autre type d'additif pour la formation d'un noyau central qui est ensuite revêtu d'un polymère approprié (par exemple une résine hydrosoluble ou une résine insoluble dans l'eau). Les techniques connues de l'homme du métier seront utilisées à cet effet.

Les microcapsules ainsi obtenues sont ensuite éventuellement formulées dans des unités de dosage appropriées.

Une administration par voie oculaire peut également être envisagée, en particulier dans le cas d'un traitement d'une lésion oculaire.

La composition pharmaceutique décrite ici se présente alors sous la forme d'une composition ophtalmique pour administration locale dans l'oeil, par exemple comme un collyre, ou une crème ophtalmique.

La composition ophtalmique peut être une solution aqueuse comprenant de l'eau distillée, une solution saline physiologique, dans laquelle les peptides de l'invention sont dissous. Un certain nombre d'additifs peut être incorporé dans la composition ophtalmique si nécessaire par exemple des agents de tampon, des agents assurant l'isotonicité avec les larmes, des conservateurs, des épaississants, des stabilisants, des anti-oxydants, des agents ajustant le pH, des agents chélatants, etc.

Les gouttes pour les yeux sont préparées par manipulation aseptique ou la stérilisation est réalisée à une étape appropriée de la préparation.

Les crèmes ophtalmiques peuvent être préparées de manière aseptique en mélangeant le principe actif à une base usuelle. Les bases pour les crèmes ophtalmiques sont par exemple la vaseline, le jelene 50 ou le plastibase, le macrogol, etc. Des surfactants peuvent être ajoutés pour augmenter l'hydrophilie. Des additifs tels que décrits ci-dessus, par exemple les conservateurs, peuvent être ajoutés si nécessaire.

En général, pour une application ophtalmique locale, un effet satisfaisant chez l'adulte est obtenu par l'administration d'une gouttelette dans l'oeil d'une préparation contenant de 0,001 à 10 %, de préférence 0,01 à 1 % poids/volume du composé de l'invention ou d'un sel pharmaceutiquement acceptable de celui-ci plusieurs fois, de préférence une à six fois par jour, et à chaque fois avec de préférence une à quatre gouttelettes par oeil, et dans le cas d'utilisation d'une crème ophtalmique, d'une préparation contenant 0,001 à 10 %, de préférence 0,01 à 1 % poids/volume du composé de l'invention ou d'un sel pharmaceutiquement acceptable de celui-ci, avec une application de préférence une à six fois par jour dans l'oeil.

Les peptides, molécules chimères et acides nucléiques selon l'invention peuvent également être formulés sous la forme de liposomes. Les liposomes sont formés à partir de phospholipides qui sont dispersés dans un milieu aqueux et forment spontanément des vésicules bicouches concentriques multilamellaires. Ces vésicules ont généralement un diamètre de 25 nm à 4 µm et peuvent être soniquées, conduisant à la formation de vésicules plus petites unilamellaires, de diamètre de 200 à 500 Å, contenant une solution aqueuse en leur coeur.

Les liposomes peuvent être particulièrement avantageux pour administrer le médicament à une cible cellulaire ou tissulaire précise. Pour cela, les lipides peuvent être couplés chimiquement à des molécules de ciblage, tels que des peptides de ciblage (par exemple hormones) ou des anticorps.

Les exemples et figures suivants illustrent l'invention sans en limiter la portée.

### Description des figures

La **Figure 1A** présente des histogrammes représentant le nombre de cellules d'adénocarcinome prostatique PC-3 (nombre indiqué en ordonnées x 10⁴) par puits en fonction de la concentration en dermaseptine B2 (en µg/ml) utilisée lors des traitements. ** : p < 0,01 versus contrôle (0). *** : p < 0,001 versus contrôle (0).
La **Figure 1B** présente des histogrammes représentant le nombre de cellules embryonnaires de souris CES (nombre indiqué en ordonnées x 10⁴) par puits en fonction de la concentration en dermaseptine B2 (en µg/ml) utilisée lors des traitements. * : p < 0,05 versus contrôle (0). *** : p < 0,001 versus contrôle (0).
La **Figure 1C** présente des histogrammes représentant le nombre de cellules d'hyperplasie prostatique G1947 (nombre indiqué en ordonnées x 10⁴) par puits en fonction de la concentration en dermaseptine B2 (en µg/ml) utilisée lors des traitements. * : p < 0,05 versus contrôle (0).
La **Figure 2A** présente des histogrammes représentant le nombre de cellules de lymphomes humains LB-EBV (nombre indiqué en ordonnées x 10⁴) par puits en fonction de la concentration en dermaseptine B2 (en µM) utilisée lors des traitements. ** : p < 0,01 versus contrôle (C). *** : p < 0,001 versus contrôle (C).
La **Figure 2B** présente des histogrammes représentant le nombre de cellules de lymphomes humains Raji (nombre indiqué en ordonnées x 10⁴) par puits en fonction de la concentration en dermaseptine B2 (en µM) utilisée lors des traitements. *** : p < 0,001 versus contrôle (C).
La **Figure 3A** présente des histogrammes représentant le nombre de colonies de cellules d'adénocarcinome humain PC-3 par mm³ traitées avec 5 µM de dermaseptine B2 ou non traitées (C). *** : p < 0,001 versus contrôle (C).
La **Figure 3B** présente des histogrammes représentant le nombre de colonies de cellules de carcinome humain MBA-MB231 par mm³ traitées avec 5 µM de dermaseptine B2 ou non traitées (C). *** : p < 0,001 versus contrôle (C).
La **Figure 4A** présente un graphique représentant l'évolution au cours du temps du volume tumoral (en mm³) de tumeurs obtenues par xénogreffes de cellules PC-3 à des souris athymiques. Les souris ont été traitées avec du PBS (■), du Taxol (●) ou de la dermaseptine B2 (▼), les traitements commençant une semaine après l'injection des cellules. Les flèches indiquent les différentes posologies du traitement par la dermaseptine B2.
La **Figure 4B** présente un graphique représentant le poids (en g) des tumeurs obtenues par xénogreffes de cellules PC-3 à des souris athymiques, après 29 jours de traitement. Les souris ont été traitées avec du PBS (■), du Taxol (▲) ou de la dermaseptine B2 (▼), les traitements commençant une semaine après l'injection des cellules.
La **Figure 5A** présente des histogrammes représentant le nombre de cellules ABAE (en pourcentage) par puits en fonction de la concentration en dermaseptine B2 (en µM) utilisée lors des traitements. *** : p < 0,001 versus contrôle (0).
La **Figure 5B** présente des histogrammes représentant le nombre de capillaires par champ formés par des cellules ABAE en présence de FGF-2 traitées avec 5 µM de dermaseptine B2 ou non traitées (C). *** : p < 0,001 versus contrôle (C).
La **Figure 6** présente des histogrammes représentant le pourcentage de cellules PC-3, incubées en absence (C) ou en présence (D) de 2,5 µM de dermaseptine B2 pendant 24 h ou 72 h, positives à la fois au marquage à l'Annexine V et au marquage à l'iodure de propidium.
La **Figure 7** présente des histogrammes représentant le pourcentage de cellules PC-3, incubées en absence (C) ou en présence (D) de 2,5 µM de dermaseptine B2 pendant 24 h ou 72 h, négatives à la fois au marquage à l'Annexine V et au marquage à l'iodure de propidium.
La **Figure 8** présente des histogrammes représentant le pourcentage de cellules PC-3, incubées en absence (C) ou en présence (D) de 2,5 µM de dermaseptine B2 pendant 24 h ou 72 h, positives au marquage à l'Annexine V mais négatives au marquage à l'iodure de propidium.
La **Figure 9** présente des histogrammes représentant le pourcentage de cellules PC-3, incubées en absence (C) ou en présence (D) de 2,5 µM de dermaseptine B2 pendant 24 h ou 72 h, négatives au marquage à l'Annexine V mais positives au marquage à l'iodure de propidium.
La **Figure 10** présente l'analyse Dot Blot en cytométrie en flux des cellules PC-3 non traitées marquées à l'Annexine V FITC et à l'iodure de propidium (PI).
La **Figure 11** présente l'analyse Dot Blot en cytométrie en flux des cellules PC-3 traitées pendant 24 h par 2,5 µM de dermaseptine B2, marquées à l'Annexine V FITC et à l'iodure de propidium (PI).

### Exemples

Les exemples suivants démontrent la capacité de la dermaseptine B2 à inhiber la prolifération cellulaire, et par conséquent l'intérêt de son utilisation dans le traitement de désordres prolifératifs.

### Exemple 1: La dermaseptine B2 inhibe la croissance des cellules tumorales adhérentes ou non-adhérentes mais n'a pas que peu d'effet sur des cellules non tumorales.

L'activité anti-proliférative de la dermaseptine B2 a été évalué *in vitro* sur différents types de cellules: des cellules d'adénocarcinome PC-3 et d'hyperplasie G1947 prostatique humain ainsi que des cellules embryonnaires de souris (CES). Les différents types de cellules ont été ensemencés dans des plaques 24 puits à 10⁴ cellule/cm² dans 0,5 ml de milieu de culture (RPMI supplémenté de 5 % de sérum de veau foetal pour les PC-3 et G1947 ou DMEM supplémenté de 10% de sérum de veau foetal pour les CES). Après 24 heures, les cellules ont été traitées avec différentes doses de dermaseptine B2. Le traitement a ensuite été renouvelé aux jours 3 et 5 après l'ensemencement des cellules. Le 6^{éme} jour, le dénombrement cellulaire a été estimé par coloration au cristal violet. Les cellules ont été rincées au PBS, fixées sur le plastique par déshydratation à l'éthanol absolu, puis colorées par une solution de cristal violet à 0,2 % dans l'éthanol 2 % pendant 15 min. Après lavage, les cellules sont solubilisées par une solution de SDS 1 %. La densité optique (DO) du cristal violet a été mesurée au spectrophotomètre à 595 nm. Une gamme étalon a été effectuée pour la conversion DO-nombre de cellules.

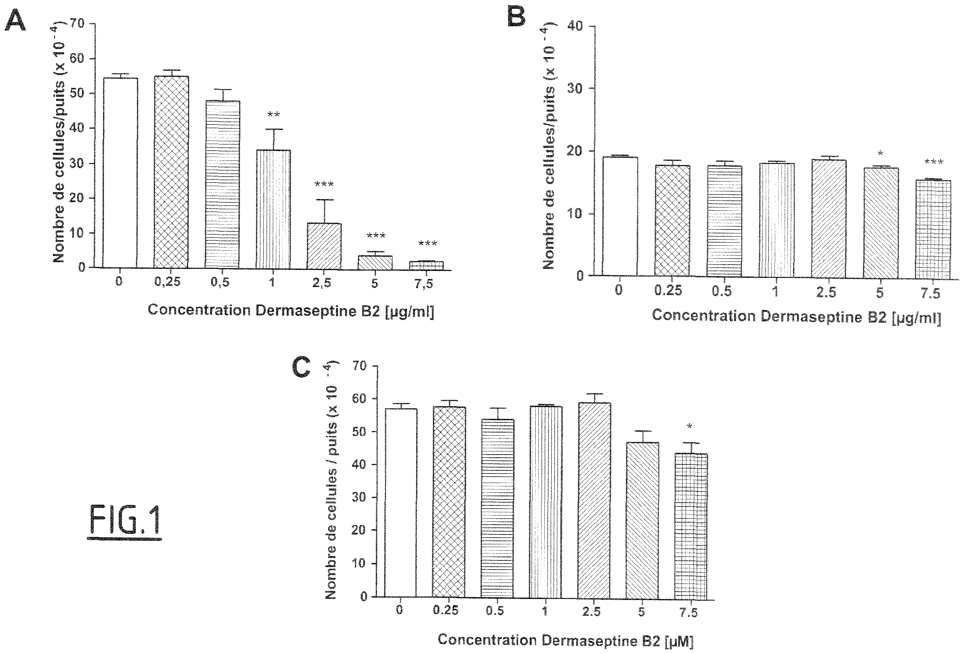

La dermaseptine B2 est capable d'inhiber de manière dose dépendante la prolifération des cellules tumorales PC-3 (**Figure 1**)**.** Cette inhibition est supérieure à 90% dès 5 µM de dermaseptine B2. A cette concentration, l'effet de la dermaseptine B2 est plutôt le reflet d'un effet cytotoxique sur ces cellules. En effet, les inventeurs ont observé que la majorité des cellules étaient pour ces concentrations décollées du fond de la boite de culture à la fin du test. Une légère inhibition d'environ 20% a été observée sur les cellules d'hyperplasie humaine G1947 pour 7,5 µM de dermaseptine B2. Cette inhibition était encore plus modeste (environ 10%) sur les cellules CES pour la dose maximum testée. Il faut noter que contrairement aux PC-3, aucun détachement cellulaire n'a été observé au cours du traitement des cellules embryonnaires et G1947.

La dermaseptine B2 a également été testée sur la croissance de deux lignées non adhérentes issues de lymphomes B humains: les lignées Raji et LB-EBV. Les cellules ont été ensemencées à 10⁴ cellules par puits dans 0,5 ml de milieu RPMI supplémenté de 2,5 % de sérum de veau foetal décomplémenté. 24 heures après l'ensemencement, les cellules ont été traitées avec différentes concentrations de dermaseptine B2. Le traitement a ensuite été renouvelé aux jours 3 et 5 après l'ensemencement des cellules. Le 7éme jour, le dénombrement cellulaire a été estimé par coloration au cristal violet comme décrit précédemment.

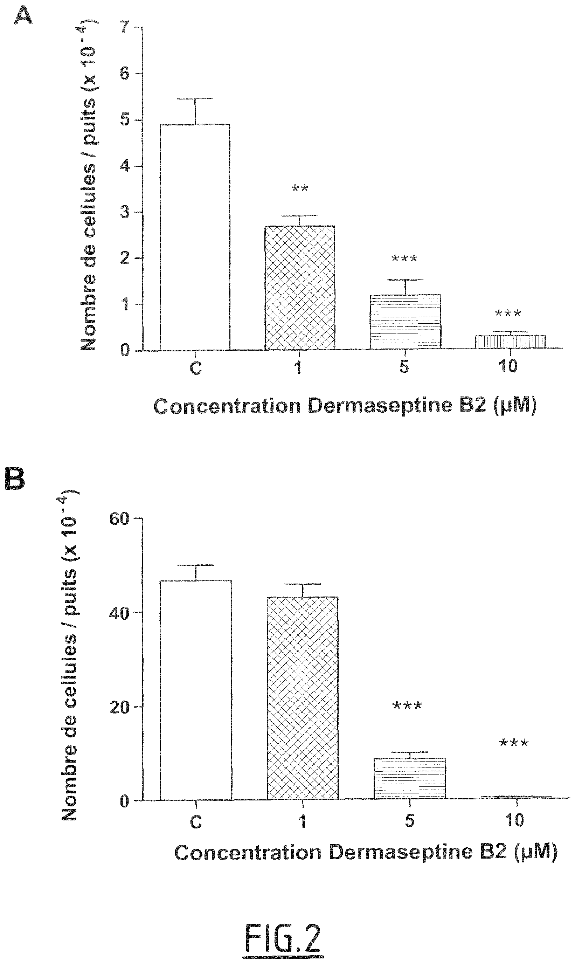

La dermaseptine B2 inhibe aussi de manière dose dépendante la prolifération des deux lignées étudiées de lymphomes humains (**Figure 2**). A 10 µM la dermaseptine B2 inhibe d'environ 95% la croissance des cellules LB-EBV et Raji.

### Exemple 2: La dermaseptine B2 inhibe la croissance des cellules tumorales cultivée in vitro en agar mou.

Afin de confirmer l'inhibition de la croissance cellulaire observée sur plastique avec les cellules PC-3, les inventeurs ont testé l'effet de la dermaseptine B2 sur la capacité des cellules PC-3 à se multiplier de manière indépendante de l'ancrage en formant des colonies dans l'agar. Pour ce test, les cellules ont été ensemencées à une densité de 2,5x10³ cellules par cm² dilué dans du milieu de culture complet (RPMI supplémenté de 5% de sérum de veau foetal) contenant 0,35% d'agar et des concentrations variables de dermaseptine B2, dans des boites de 12 puits contenant 1 ml d'agar à 0,6% solidifié. Les mêmes concentrations variables de dermaseptine B2 ont également été ajoutées dans le milieu de culture complet déposé au dessus des cultures le jour de l'ensemencement ainsi que 2 fois par semaine. Après une incubation de 12 jours à 37°C dans un incubateur saturé en vapeur d'eau et contenant 7% CO₂, les colonies ayant un diamètre supérieur à 50 µm ont été dénombrées. Chaque mesure a été effectuée en triple et chaque expérience a été répétée trois fois.

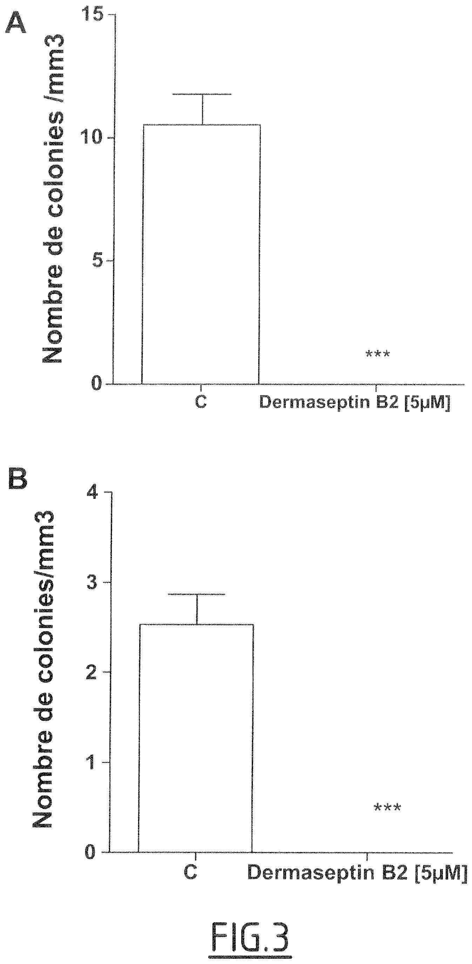

A une concentration de 5 µM la dermaseptine B2 inhibe totalement la croissance des cellules PC-3 sur agar mou (**Figure 3A**). Au jour 5, dans les puits traités avec la dermaseptine B2, aucune cellule subsiste.

Cette inhibition de la prolifération reflète un effet de la dermaseptine B2 sur l'induction de la mort cellulaire ou un effet cytotoxique aux doses utilisés. En effet dés le troisième jour après leur ensemencement, les cellules traitées avec la dermaseptine n'apparaissaient pas réfringentes en observation sous le microscope à contraste de phase comme les cellules non traitées. Lorsque cette expérience a été réalisée avec une lignée de carcinomes mammaire humain MBA-MB231, des résultats similaires ont été observés (**Figure 3B**).

### Exemple 3: La dermaseptine B2 inhibe la croissance tumorale des cellules PC-3 in vivo dans un modèle de souris nude.

Ayant établi que la dermaseptine B2 a la capacité d'inhiber la croissance sur agar des cellules PC-3 et MDA-MB231, les inventeurs ont testé l'effet de ce peptide sur la croissance des tumeurs induites par injection de PC-3 à des souris nude. Des lots de 10 souris nude (nude/nude, Laboratoire IFFA CREDO) ont été injectées avec 2x10⁶ cellules PC-3. Une semaine après l'injection des cellules, les animaux présentant une tumeur palpable ont été répartis aléatoirement par cage pour être traités par injection au niveau de la tumeur par 100 µl par jour d'une solution de PBS (lot contrôle) ou par une solution de dermaseptine B2 dilué dans du PBS. La dermaseptine B2 a été injectée à une concentration de 5 mg/kg la première semaine de traitement puis cette dose a été ramenée à 0,5 mg/kg pendent 12 jours. Suite à une reprise de croissance tumorale chez certains animaux traités, la dermaseptine B2 a de nouveau été injectée à 5 mg/kg pendant une semaine puis la dose a été ramenée à 2 mg/kg jusqu'à la fin du traitement. En contrôle, un lot de souris a également été traité avec du Taxol® (paclitaxel, Bristol-Myers Squibb) deux fois par semaine en injection intra-périnotéale à 10 mg/kg. La taille des tumeurs a été mesurée à l'aide d'un pieds à coulisse deux fois par semaine jusqu'au sacrifice des animaux 29 jours après le début du traitement. Après sacrifice, les tumeurs ont été prélevées et pesées.

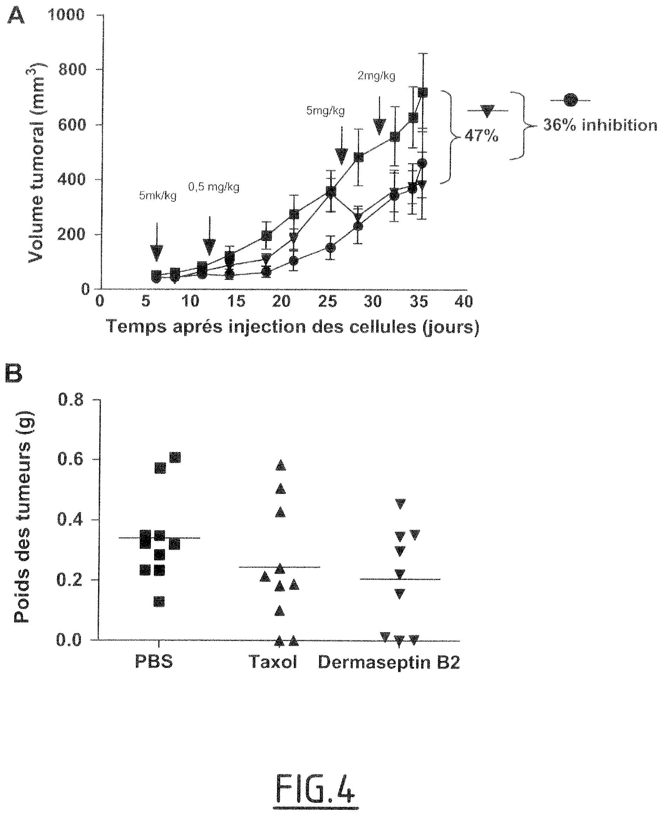

Les résultats sont présentés sur la **Figure 4A** et indiquent que la dermaseptine B2 induit une inhibition de la croissance des tumeurs de 47% comparée à celle des tumeurs non traitées. Cette inhibition est sensiblement plus forte que celle observée avec le Taxol® qui n'est que de 36%.

Après sacrifice des animaux, les tumeurs ont été prélevées et pesées. La **Figure 4B** montre les différences de poids des tumeurs prélevés sur les animaux contrôles et traités soit par la dermaseptine B2 soit par le Taxol. Les résultats obtenus confirment ceux obtenus sur les mesures des volumes tumoraux au cours des traitements, notamment une disparition totale de tumeur chez 3 souris traitées par la dermaseptine B2.

### Exemple 4: La dermaseptine B2 inhibe la croissance de cellules endothéliales et la formation de pseudo-capillaires in vitro.

L'activité angiostatique de la dermaseptine B2 a été évaluée sur ses capacités à inhiber d'une part la prolifération de cellules endothéliales aortiques de boeuf (ABAE) sur plastique et d'autre part la formation de pseudo capillaires par ces mêmes cellules en gel de collagène. Pour le test de prolifération, les cellules ABAE on été ensemencées en plaques 24 puits à une densité de 10⁴ cellules/puits en milieu DMEM supplémenté de 10% de sérum de veau foetal et de 5 ng/ml de FGF-2. Comme pour les tests de prolifération précédemment décrits, 24 h après l'ensemencement, les cellules ont été traitées avec différentes doses de dermaseptine B2. Le traitement a ensuite été renouvelé aux jours 3 et 5 après l'ensemencement des cellules. Le 7éme jour, le dénombrement cellulaire a été estimé par coloration au cristal violet.

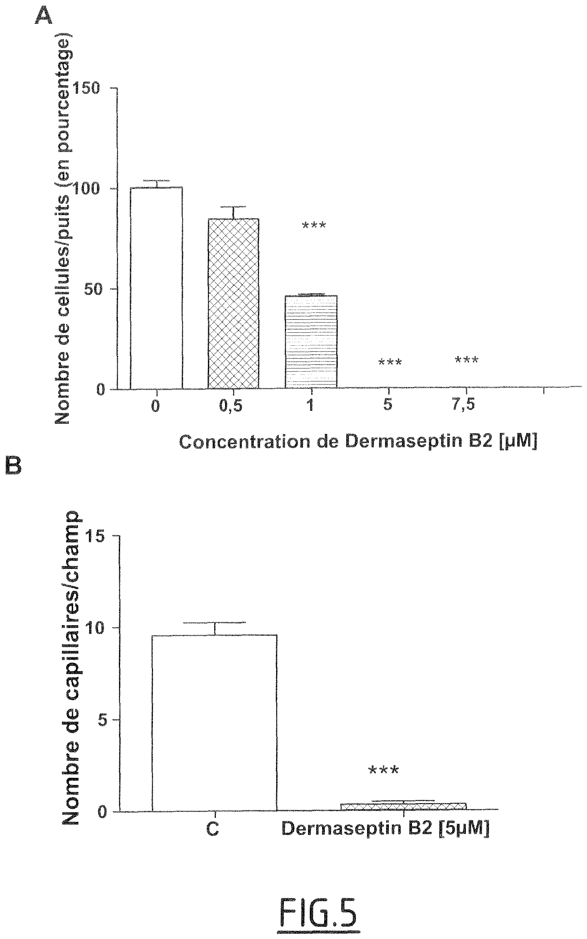

Les inventeurs ont montré que la dermaseptine B2 induisait une inhibition dose dépendante et totale de la croissance des cellules ABAE (**Figure 5A**). Comme pour les cellules PC-3, l'effet obtenu semble lié à une cytotoxicité ou une induction de la mort cellulaire de la dermaseptine B2 sur les ABAE puisque pour les doses de 5 et 7,5 µM, les inventeurs ont observé une augmentation de la présence de cellules mortes (un détachement cellulaire observé au cours du traitement).

L'effet de la dermaseptine B2 sur la formation de pseudos capillaires a été réalisé par utilisation du test de Montessano. Ce test permet d'évaluer la différenciation de cellules ABAE en pseudo capillaires lorsqu'elles sont ensemencées en mono couche sur un gel de collagène I. Pour ce test des cellules ABAE ont été ensemencées en plaque de 24 puits sur mono couche de collagène I à raison de 10⁵ cellules/puits dans un milieu DMEM supplémenté de 10% de sérum de veau foetal. 24 heures après l'ensemencement, 20 ng/ml de FGF-2 ont été ajoutés aux cellules en absence ou présence de concentrations variables de dermaseptine B2. Ce traitement a été renouvelé pendant deux jours et la formation d'un réseau de pseudo capillaire a été appréciée 24 heures plus tard par observation au microscope à contraste de phase en mesurant le nombre de capillaires formés par champs d'observation (**Figures 5C et D**).

La **Figure 5B** montre que la dermaseptine B2 utilisée ici à 5 µM inhibe fortement la formation de pseudo capillaires induite par le FGF-2. Il faut noter que contrairement au test de prolifération, la dermaseptine B2 n'a pas eu d'effet cytotoxique sur la mono couche de cellules ABAE.

L'ensemble des résultats présentés indiquent que la dermaseptine B2 est capable d'inhiber deux étapes essentielles de l'angiogenèse : la prolifération ainsi que la différenciation des cellules endothéliales.

### Exemple 5: La dermaseptine B2 augmente l'apoptose et la nécrose chez les cellules d'adénocarcinome humain PC3.

Lors des tests de prolifération des PC3 ou des ABAE, en présence de dermaseptine B2, l'effet inhibiteur rapidement observé ainsi que la non réfringence des cellules traitées laissaient supposer que la dermaseptine B2 pourrait exercer un rôle cytotoxique ou d'induction de la mort cellulaire plutôt que de bloquer la croissance des cellules sensibles. Afin de déterminer si la dermaseptine B2 pouvait induire l'apoptose des cellules sensibles, les inventeurs ont réalisé des expériences de double marquage Annexine V et iodure de propidium sur des cellules PC3 en culture sur plastique et traitées ou non par la dermaseptine B2. Ces expériences ont été suivies par analyse des cellules marquées en cytométrie de flux.

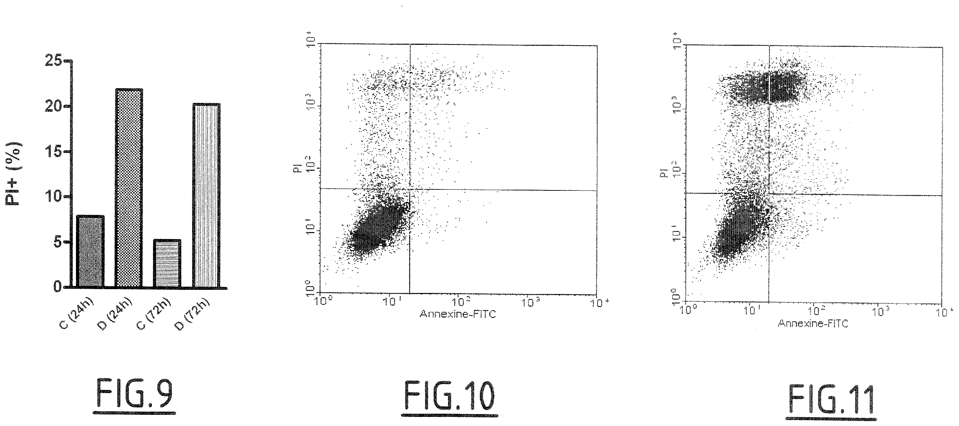

Pour ceci les cellules ont été ensemencées en plaque 6 puits en absence ou présence de 2,5 µM de dermaseptine B2 pendant 24 ou 72 heures. Les cellules ont ensuite été décollées, lavées avec du PBS froid et resuspendues dans 100 µl de tampon de fixation (MACs, Miltenyi Biotec) contenant de l'Annexine V FITC pendant 10 min dans l'obscurité. Après des lavages en PBS, les cellules ont de nouveau été marquées par incubation dans un tampon de fixation contenant une concentration finale de 1 µg/ml d'iodure de propidium (PI) pendant 5 min dans l'obscurité. Après de nouveau lavages, les cellules ont été analysées par passage dans un cytomètre de flux (FACScan, Becton Dickinson Labware) (**Figures 10** et 11).

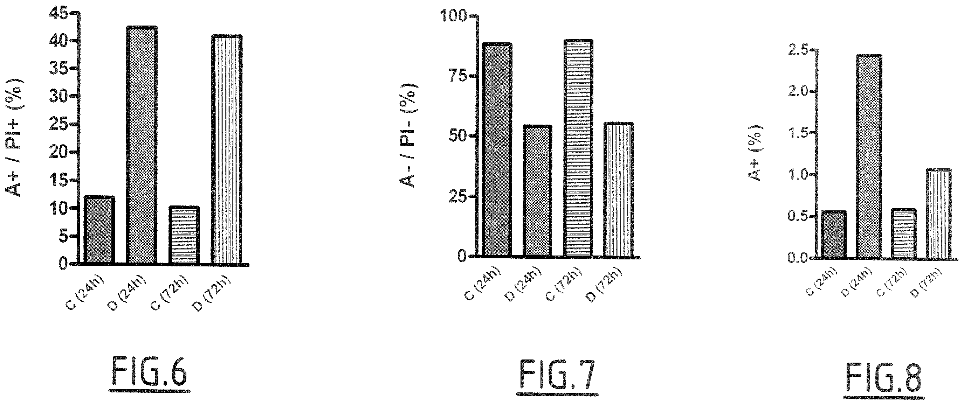

Un traitement de 24 h des cellules PC3 avec 2,5 µM de dermaseptine B2 induit une forte augmentation (environ 30%) des cellules doublement marquées à l'Annexine V et au PI, traduisant une augmentation de l'apoptose cellulaire (**Figure 6**). Ce pourcentage n'évolue pas si les cellules sont traitées 72 h. A l'inverse le pourcentage de cellules non marquées chute brutalement dans les mêmes proportions (**Figure 7**). La dermaseptine B2 provoque également une augmentation de 5 fois le pourcentage de cellules uniquement marquées à l'Annexine V après 24 h de traitement, représentant ainsi les cellules entrant en apoptose précoce (**Figure 8**). Enfin la dermaseptine B2 induit également une augmentation de 3 fois le pourcentage de cellules marquées uniquement au PI, traduisant ainsi une mort cellulaire rapide synonyme de nécrose (**Figure 9**). Là encore, peu de modifications ont été observées entre 24 et 72 h de traitement.

Ces résultats démontrent que l'effet de la dermaseptine B2 sur les PC3 se traduit par une forte augmentation de la mort cellulaire dés 24h de traitement.

## Revendications

1. Peptide isolé comprenant, ou consistant en, une séquence d'acides aminés choisie dans le groupe constitué de
- la séquence de la dermaseptine B2, la séquence du précurseur de la dermaseptine B2;
- une séquence d'acides aminés ayant au moins 80% d'identité avec les séquences de la dermaseptine B2 ou du précurseur de la dermaseptine B2; et
- un fragment de ces séquences;
sous réserve que le peptide isolé inhibe la croissance et/ou la prolifération cellulaire,
pour son utilisation pour inhiber la croissance et/ou la prolifération cellulaire dans le traitement ou la prévention d'une tumeur.

2. Peptide pour l'utilisation selon la revendication 1, ledit peptide comprenant, ou consistant en, une séquence d'acides aminés choisie dans le groupe constitué
- des séquences SEQ ID NO: 1 et SEQ ID NO: 2;
- une séquence d'acides aminés ayant au moins 80% d'identité avec les séquences SEQ ID NO: 1 ou SEQ ID NO: 2; et
- un fragment de ces séquences;
sous réserve que le peptide isolé inhibe la croissance et/ou la prolifération cellulaire.

3. Peptide pour l'utilisation selon la revendication 1 ou 2, ledit peptide consistant en une séquence d'acides aminés choisie dans le groupe constitué des séquences SEQ ID NO: 1 et SEQ ID NO: 2.

4. Peptide pour l'utilisation selon l'une quelconque des revendications 1 à 3, ledit peptide comportant une modification chimique améliorant sa stabilité et/ou sa biodisponibilité.

5. Molécule chimère comprenant au moins un peptide tel que défini dans l'une quelconque des revendications 1 à 4, dans laquelle ledit peptide est lié à :
a) un composé thérapeutique utile pour le traitement de désordres prolifératifs;
b) une enzyme capable de convertir une molécule en un composé thérapeutique utile pour le traitement de désordres prolifératifs, l'enzyme étant sélectionnée parmi les membres de la famille des métalloprotéinases matricielles, l'urokinase ou la plasmine; ou
c) une molécule porteuse,
pour son utilisation pour inhiber la croissance et/ou la prolifération cellulaire dans le traitement ou la prévention d'une tumeur.

6. Peptide pour l'utilisation selon l'une quelconque des revendications 1 à 4 ou molécule chimère pour l'utilisation selon la revendication 5, où ladite tumeur est choisie parmi une tumeur solide, une leucémie, une métastase tumorale, une tumeur bénigne, un hémangiome et un adénome hépatocellulaire.

7. Acide nucléique comprenant, ou consistant en, une séquence codant pour un peptide tel que défini dans l'une des revendications 1 à 4, pour son utilisation pour inhiber la croissance et/ou la prolifération cellulaire dans le traitement ou la prévention d'une tumeur.

8. Vecteur comprenant un acide nucléique selon la revendication 7, dans lequel ledit acide nucléique est lié de manière fonctionnelle à un ou des élément(s) permettant l'expression dudit peptide, pour son utilisation pour inhiber la croissance et/ou la prolifération cellulaire dans le traitement ou la prévention d'une tumeur.

9. Acide nucléique pour l'utilisation selon la revendication 7 ou vecteur pour l'utilisation selon la revendication 8, où ladite tumeur est choisie parmi une tumeur solide, une leucémie, une métastase tumorale, une tumeur bénigne, un hémangiome et un adénome hépatocellulaire.

10. Peptide tel que défini dans l'une des revendications 1 à 4 ou molécule chimérique telle que définie dans la revendication 5, en combinaison avec un second composé thérapeutique utile pour le traitement de tumeurs, pour leur utilisation dans le traitement ou la prévention d'une tumeur.

## Patentansprüche

1. Isoliertes Peptid, das eine Aminosäuresequenz umfasst oder daraus besteht, die aus der Gruppe ausgewählt ist, die besteht aus
- der Sequenz des Dermaseptin B2, der Sequenz der Vorstufe des Dermaseptin B2;
- einer Aminosäuresequenz mit mindestens 80 % Identität mit den Sequenzen des Dermaseptin B2 oder der Vorstufe des Dermaseptin B2; und
- einem Fragment dieser Sequenzen;
unter dem Vorbehalt, dass das isolierte Peptid das Zellwachstum und/oder die Zellvermehrung inhibiert,
für seine Verwendung zum Inhibieren des Zellwachstums und/oder der Zellvermehrung bei der Behandlung oder Verhinderung eines Tumors.

2. Peptid für die Verwendung nach Anspruch 1, wobei das Peptid eine Aminosäuresequenz umfasst oder daraus besteht, die aus der Gruppe ausgewählt ist, die besteht aus
- den Sequenzen SEQ ID NR.: 1 und SEQ ID NR.: 2;
- einer Aminosäuresequenz mit mindestens 80 % Identität mit den Sequenzen SEQ ID NR.: 1 oder SEQ ID NR.: 2; und
- einem Fragment dieser Sequenzen;
unter dem Vorbehalt, dass das isolierte Peptid das Zellwachstum und/oder die Zellvermehrung inhibiert.

3. Peptid für die Verwendung nach Anspruch 1 oder 2, wobei das Peptid aus einer Aminosäuresequenz besteht, die aus der Gruppe ausgewählt ist, die aus den Sequenzen SEQ ID NR.: 1 und SEQ ID NR.: 2 besteht.

4. Peptid für die Verwendung nach einem der Ansprüche 1 bis 3, wobei das Peptid eine chemische Modifikation umfasst, die seine Stabilität und/oder seine Bioverfügbarkeit verbessert.

5. Chimeres Molekül mit mindestens einem Peptid, wie in einem der Ansprüche 1 bis 4 definiert, wobei das Peptid gebunden ist an:
a) eine therapeutische Nutzverbindung für die Behandlung von proliferativen Störungen;
- ein Enzym, das ein Molekül in eine therapeutische Nutzverbindung für die Behandlung von proliferativen Störungen umwandeln kann, wobei das Enzym aus den Mitgliedern der Familie der Matrixmetalloproteinasen, der Urokinase oder des Plasmins ausgewählt ist; oder
- ein Trägermolekül,
für seine Verwendung zum Inhibieren des Zellwachstums und/oder der Zellvermehrung bei der Behandlung oder Verhinderung eines Tumors.

6. Peptid für die Verwendung nach einem der Ansprüche 1 bis 4 oder chimeres Molekül für die Verwendung nach Anspruch 5, wobei der Tumor aus einem festen Tumor, einer Leukämie, einer Tumormetastase, einem gutartigen Tumor, einem Hemangiom und einem hepatozellulären Adenom ausgewählt ist.

7. Nukleinsäure, die eine Sequenz umfasst oder daraus besteht, die ein Peptid, wie in einem der Ansprüche 1 bis 4 definiert, codiert, für ihre Verwendung zum Inhibieren des Zellwachstuns und/oder der Zellvermehrung bei der Behandlung oder der Verhinderung eines Tumors.

8. Vektor mit einer Nukleinsäure nach Anspruch 7, wobei die Nukleinsäure in funktionaler Weise an ein oder Elemente gebunden ist, die die Expression des Peptids ermöglichen, für seine Verwendung zum Inhibieren des Zellwachstums und/oder der Zellvermehrung bei der Behandlung oder Verhinderung eines Tumors.

9. Nukleinsäure für die Verwendung nach Anspruch 7 oder Vektor für die Verwendung nach Anspruch 8, wobei der Tumor aus einem festen Tumor, einer Leukämie, einer Tumormetastase, einem gutartigen Tumor, einem Hemangiom und einem hepatozellulären Adenom ausgewählt ist.

10. Peptid, wie in einem der Ansprüche 1 bis 4 definiert, oder chimeres Molekül, wie in Anspruch 5 definiert, in Kombination mit einer zweiten therapeutischen Nutzverbindung für die Behandlung von Tumoren, für ihre Verwendung bei der Behandlung oder der Verhinderung eines Tumors.

## Claims

1. Isolated peptide comprising, or consisting of, a sequence of amino acids selected from the group consisting of
- the sequence of dermaseptin B2, the sequence of the precursor of dermaseptin B2;
- a sequence of amino acids having at least 80% identity with the sequences of dermaseptin B2 or the precursor of dermaseptin B2; and
- a fragment of these sequences;
provided that the isolated peptide inhibits cell growth and/or proliferation,
for use for inhibiting cell growth and/or proliferation in the treatment or prevention of a tumour.

2. The peptide for the use according to claim 1, said peptide comprising, or consisting of, a sequence of amino acids selected from the group consisting of
- sequences SEQ ID NO: 1 and SEQ ID NO: 2;
- a sequence of amino acids having at least 80 % identity with sequences SEQ ID NO: 1 or SEQ ID NO: 2; and
- a fragment of these sequences;
provided that the isolated peptide inhibits cell growth and/or proliferation.

3. The peptide for the use according to claim 1 or 2, said peptide consisting of a sequence of amino acids selected from the group consisting of sequences SEQ ID NO: 1 and SEQ ID NO: 2.

4. The peptide for the use according to any of claims 1 to 3, said peptide comprising a chemical modification improving its stability and/or its bioavailability.

5. A chimeric molecule comprising at least one peptide as defined in any of claims 1 to 4, wherein said peptide is linked to:
a) a therapeutic compound useful for the treatment of proliferative disorders;
b) an enzyme capable of converting a molecule into a therapeutic compound useful for the treatment of proliferative disorders, the enzyme being selected from the members of the matrix metalloproteinase family, urokinase or plasmin; or
c) a carrier molecule,
for use for inhibiting cell growth and/or proliferation in the treatment or prevention of a tumour.

6. The peptide for the use according to any of claims 1 to 4 or a chimeric molecule for the use according to claim 5, wherein said tumour is selected from a solid tumour, a leukaemia, a tumour metastasis, a benign tumour, a haemangiomas and a hepatocellular adenoma.

7. A nucleic acid comprising, or consisting of, a sequence coding for a peptide as defined in one of claims 1 to 4, for the use for inhibiting cell growth and/or proliferation in the treatment or prevention of a tumour.

8. A vector comprising a nucleic acid according to claim 7, wherein said nucleic acid is functionally linked to one or more element(s) allowing the expression of said peptide, for the use for inhibiting cell growth and/or proliferation in the treatment or prevention of a tumour.

9. The nucleic acid for the use according to claim 7 or vector for the use according to claim 8, wherein said tumour is selected from a solid tumour, a leukaemia, a tumour metastasis, a benign tumour, a haemangiom and a hepatocellular adenoma.

10. The peptide as defined in one of claims 1 to 4 or a chimeric molecule as defined in claim 5, in combination with a second therapeutic compound useful for the treatment of tumours, for the use in the treatment or prevention of a tumour.
